# EUROPÄISCHE PATENTANMELDUNG

(11) **EP 1 288 647 A2**
(43) Veröffentlichungstag der Anmeldung: **05.03.2003**
(21) Anmeldenummer: 02017777.0
(22) Anmeldetag: 12.08.2002
(51) Int. Cl.: G01N 21/03, B01L 3/00

(54) **Spektroskopisches Testsystem auf Basis von Mikrokapillaren**

(30) Priorität: 24.08.2001 DE 10140680
(71) Anmelder: BAYER AG, 51368 Leverkusen (DE)
(72) Erfinder: Hildenbrand, Karlheinz, Dr., 47802 Krefeld (DE); Dorn, Ingmar Dr., 50733 Köln (DE); Diessel, Edgar, Dr., 51061 Köln (DE); Lison, Frank, Dr., 40764 Langenfeld (DE)

(57) **Zusammenfassung**

Die Erfindung betrifft die Verwendung von spektroskopischen Methoden zur Bestimmung von Analyten in einer Probe sowie ein Testsystem zur qualitativen und/oder quantitativen Bestimmung eines oder mehrerer Analyten in einer flüssigen Probe.

## Beschreibung

Die Erfindung betrifft die Verwendung von spektroskopischen Methoden zur Bestimmung von Analyten in einer Probe sowie ein Testsystem zur qualitativen und/oder quantitativen Bestimmung eines oder mehrerer Analyten in einer flüssigen Probe.

Testsysteme zur Selbstdiagnose im home-user-Bereich, insbesondere von Blutzucker, sind seit vielen Jahren etablierter Stand der Technik.

Dennoch ist eine ständige Weiterentwicklung der bestehenden Produkte anzustreben mit dem Ziel, weitere Verbesserungen in der Genauigkeit und Reproduzierbarkeit sowie insbesondere in der Handhabung und Benutzerfreundlichkeit zu erzielen. Stand der Technik sind Biosensoren, basierend auf elektrochemischen Nachweisreaktionen, sowie Teststreifen auf Membranbasis bei denen Farbreaktionen reflektometrisch ausgewertet werden.

Im Hinblick auf Benutzerfreundlichkeit werden insbesondere Biosensoren, die über sogenannten "sip in" Mechanismen das Patientenblut einsaugen, günstig beurteilt.

Um reproduzierbare Ergebnisse zu erhalten, muss bei den elektrochemischen Sensoren (siehe z. B. USP 5 759 364) das gesamte Elektrodenkompartiment mit Blut bedeckt sein, wozu bei den bisher bekannten Produkten in der Regel mindestens 3 µl Blut oder mehr benötigt werden.

Als Teststreifen formatierte Testsysteme auf Membranbasis (z. B. USP 5 453 360), die reflektometrisch ausgewertet werden, erfordern in der Regel noch höhere Blutmengen.

Im Hinblick auf Reproduzierbarkeit und Präzision sind neben der Konstanz der Sensorgeometrie bzw. Membrammorphologie vor allem die gleichmäßige Applikation des biochemischen Reagenzsystems in das Testelement entscheidend.

Bei den elektrochemischen Biosensoren handelt es sich hierbei um die Aufbringung einer Enzym/Mediator-Rezeptur, beispielsweise per Siebdruck oder Mikropipetting auf das Sensor-Elektrodenkompartiment.

Bei den colorimetrischen Teststreifensysteme werden mikroporöse Membranen mit Enzym/Indikator-Rezepturen getränkt bzw. beschichtet.

Mikrokapillaren in zahlreichen Variationen sind in Form von Säulen aus der Chromatographie, insbesondere der Gaschromatographie (GC) und der Kapillarelektrophorese (CE), bekannt ("Making and Manipulating Capillary Columns for Gas Chromatography" von Kurt Grob, Huethig Verlag, 1986) und werden nachfolgend als Mikrokapillarsäulen bezeichnet. Mikrokapillarsäulen sind in Längen von bis zu 50 oder sogar 100 m in hoher Reproduzierbarkeit herstellbar.

Die nachveröffentlichte deutsche Patentanmeldung 100 08 906 offenbart, dass auf Basis von Mikrokapillarensäule bei der colorimetrischen Bestimmung von Analyten wie Glucose in flüssigen Proben wie Blut deutliche Fortschritte hinsichtlich der o. g. Zielkriterien erreicht werden können. Die colorimetrische Bestimmung erfordert jedoch die Integration eines chromogenen enzymatischen Reagenzsystems in das Innere der Mikrokapillarsäule. Hierbei handelt es sich um einen relativ komplexen Prozess verbunden mit erheblichen Kosten für das enzymatische Reagenzsystem. Darüber hinaus können derartige chromogene Testsysteme nur für einen Analyten verwendet werden. Beispielsweise kann mit einem Glucoseoxidase-haltigen Reagenzsystem nur einmal Glucose nachgewiesen werden.

Aufgabe der Erfindung war es, die Bestimmung von Analyten in flüssigen Proben unter Nutzung der Vorteile der Mikrokapillaren ohne ein dazu erforderliches enzymatisches Reagenzsystem, d. h. reagenz-frei (ohne analytische Hilfsreagenzien), zu ermöglichen.

Gegenstand der Erfindung ist die Verwendung von spektroskopischen Methoden zur qualitativen und/oder quantitativen Bestimmung eines oder mehrerer Analyten in einer mit einer Mikrokapillare aufgenommenen Probe.

Gegenstand der Erfindung ist auch ein Testsystem zur qualitativen und/oder quantitativen Bestimmung eines oder mehrerer Analyten in einer flüssigen Probe, bei dem die Probenaufnahme mit einer Mikrokapillare und die Bestimmung des oder der Analyten mit einer spektroskopischen Methode erfolgt.

Erfindungsgemäß wird die Probenflüssigkeit, beispielsweise Vollblut oder interstitielle Gewebsflüssigkeit, über Kapillarkräfte in eine Mikrokapillare, wie sie beispielsweise in der Chromatographie verwendet werden, eingesaugt. Das hat neben dem Aspekt der kleinen Probenvolumina auch den Vorteil, dass die Probenflüssigkeit nicht mit Bindemitteln oder Klebstoffen, die üblicherweise zur Herstellung von konventionellen Teststreifen- oder Biosensorsystemen benötigt werden, in Kontakt kommen kann.

Geeignete spektroskopische Methoden sind dem Fachmann vertraut und werden von ihm unter Berücksichtigung der Eigenschaften von Analyt und flüssiger Probe in geeigneter Weise gewählt. Bevorzugte spektroskopische Methoden sind Infrarot-(IR-) und Raman-Spektroskopie. Besonders bevorzugte spektroskopische Methoden für den Infrarotbereich sind der Nahinfrarot (NIR)- und der mittlere Infrarotbereich (MIR). Für die Anpassung der beiden Methoden an die Analyse kleinster Probenvolumina in Mikrokapillaren kommen neben der Übertragung von Standardgeometrien verschiedene weitere Ausführungsformen in Betracht: wie z. B. oberflächensensitive Methoden wie die abgeschwächte Totalreflexion (ATR), optische Wellenleiter in Form von Lichtleitfasern oder planaroptischen Lichtwellenleitern, optische Anregung kleinster Probenvolumina mit optischen Nahfeldmethoden (SNOM), insbesondere für die Ramanspektroskopie: oberflächenverstärkte Raman-Spektroskopie (SERS) oder nichtlineare Raman-Spektroskopie wie z. B. kohärente anti-Stokes Raman-Spektroskopie (CARS), konfokale Geometrien zur Reduktion von Hintergrundsignalen. Die oberflächensensitiven Methoden wie die IR-spektroskopische ATR-Technik eignen sich besonders für Proben, die für Transmissions-Messungen ungeeignet sind.

Die Dimensionen und Materialeigenschaften der erfindungsgemäß eingesetzten Mikrokapillaren sind so beschaffen, dass sie eine im wesentlichen wässerige Probenflüssigkeit durch Kapillarkräfte einsaugen. Das eingesaugte Probevolumen ist, insbesondere für Mikrokapillaren einer Länge von etwa 1 cm, dabei vorzugsweise kleiner 3 µl, besonders bevorzugt kleiner 1 µl, ganz besonders bevorzugt 0,5 µl oder kleiner.

Geeignete Mikrokapillaren sind insbesondere aus der Gaschromatographie (GC) und der Kapillarelektrophorese (CE) bekannt. Solche Mikrokapillarsäulen erfüllen sowohl hinsichtlich Kapillargeometrie als auch Beschaffenheit der Kapillarinnenseite weitgehend oder sogar vollständig die erfindungsgemäß gestellten Anforderungen.

Die erfindungsgemäß eingesetzten Mikrokapillaren haben bevorzugt einen runden Querschnitt. Der Innendurchmesser der Mikrokapillaren beträgt bevorzugt weniger als 500 µm, besonders bevorzugt weniger als 250 µm, ganz besonders bevorzugt 10 µm bis 100 µm.

Die Länge der verwendeten Mikrokapillare beträgt vorzugsweise bis zu 2 cm, besonders bevorzugt bis zu 1 cm, ganz besonders bevorzugt etwa 0,5 cm.

Als für die erfindungsgemäßen Zwecke geeignete Mikrokapillaren haben sich GCoder CE-Säulen erwiesen, deren Dimensionen üblicherweise so beschaffen sind, dass bereits sehr geringe Probeflüssigkeitsmengen, beispielsweise 0,5 µl Blut oder interstitielle Gewebsflüssigkeit für einen Funktionstest bei Kapillarlängen von 1 cm oder weniger ausreichend sind.

Wegen der geringen Probevolumina erfüllen die erfindungsgemäßen Mikrokapillaren die Anforderungen an sogenannte "minimal invasive" Testkits, die für die Patienten besonders vorteilhaft, insbesondere schmerzarm sein sollen.

Die Mikrokapillaren bestehen aus einem geeigneten unter den jeweiligen Bedingungen inerten, für die Strahlung der spektroskopischen Methode transparenten Material. Beispiele hierfür sind Quarzglas und normales Glas.

Die Mikrokapillaren tragen vorzugsweise eine Innenbeschichtung, die in der Chromatographie auch als stationäre Phase bezeichnet wird. Für diese Beschichtung kommen zahlreiche Materialien in Frage, die im Prinzip von verfügbaren GC-Säulen bekannt sind. Geeignet für die erfindungsgemäßen Mikrokapillar-Testsysteme sind hydrophile und/oder polare Beschichtungsmaterialien, wie z. B. Polyethylenglykole mit verschiedenen Molekulargewichten (Carbowax®), Polyethylenglycolderivate, beispielsweise Carbowax® 4000 monostearat, und Cyanopropylpolysiloxan.

Hinsichtlich Aufbringen der stationären Phasen (Innenbeschichtung der Mikrokapillaren) sei auf von den GC-Säulen bekannte Standardverfahren verwiesen. Es stehen, wie in o.g. Literatur (K. Grob, 1986) beschrieben, zwei prinzipielle Verfahren zur Verfügung. Es handelt sich um das "Static Coating" und "Dynamic Coating". Nach diesen Verfahren können Mikrokapillaren in bis zu 50 oder sogar 100 m Länge beschichtet werden. Die Polymeren der stationären Phasen können auch, wie in o. g. Literatur beschrieben, kovalent an die Oberfläche der Quarzsäule gebunden sein.

Die konventionellen Mikroapillarsäulen bestehen üblicherweise aus dem bereits erwähnten Quarz (fused silica). Die früher verwendeten Glassäulen haben in der Chromatographie stark an Bedeutung verloren, sind aber wegen ihrer hervorragenden Transparenz für die erfindungsgemäßen Testsysteme durchaus von Bedeutung.

Die Quarzsäulen sind in der Regel auf ihrer Außenseite mit einer gelb/braunen hochtemperaturbeständigen Polymidschicht versehen. Dadurch wird die Sprödigkeit der Quarzkapillare aufgehoben und es entstehen gut handhabbare flexible Mikrokapillarsäulen, die hinsichtlich Herstellung und weiterer Verarbeitung als Rollenmaterial gehandhabt werden können.

Für die erfindungsgemäßen Testsysteme ist eine ausreichende Transparenz für die Strahlung der sprektroskopischen Methode erforderlich. Falls die farbige Polyimid-Beschichtung die spektroskopische Auswertung stört, können stattdessen zur Beschichtung auch transparente, farblose Polymere, wie z. B. Polysiloxane, Acrylpolymere, Polyvinylacetat, Polycarbonat, Polyamid oder Polyetherpolysulphon, verwendet werden. Gegebenenfalls kann auch die störende Beschichtung im entsprechenden Bereich der Mikrokapillare für die Auswertung, beispielsweise durch Laserablation, entfernt werden.

Essentielle Voraussetzung für die Mikrokapillaren als wesentlichem Bestandteil für die erfingungsgemäßen Testkits ist ihre Eigenschaft, Vollblut, interstitielle Gewebsflüssigkeit oder andere Probenflüssigkeiten, mit Hilfe von Kapillarkräften einzusaugen.

Es wurde überraschenderweise gefunden, dass Mikrokapillaren mit hydrophilen und/oder polaren Beschichtungen, z. B. mit Polyethylenglykol (Carbowax®) oder mit sogenannten polaren stationären Phasen, z. B. Cyanopropylpolysiloxan, Probenflüssigkeiten wie Blut oder interstitielle Gewebsflüssigkeit hervorragend einsaugen, d. h. dass hydrophile und/oder polare Beschichtungen das Einsaugen der Probenflüssigkeit durch Kapillarkräfte begünstigen, während mit hydrophoben Beschichtungen modifizierte Mikrokapillaren (z. B. Polysiloxan-modifizierte) diese Eigenschaft nicht zeigten.

Letztere Mikrokapillarentypen können jedoch durch Nachbehandeln mit bestimmten Tensiden dahingehend modifiziert werden.

Geeignete Tenside sind ionische, beispielsweise SDS, zwitterionische, beispielsweise Phospholipide, und nicht ionische beispielsweise Pluronic® oder Fluortenside (beispielsweise Bayowet FT 219®).

Derartige Tenside können auch, hinsichtlich weiterer Optimierung des Einsaugverhaltens, in oder auf den erwähnten hydrophilen Beschichtungen, z. B. Carbowax®, enthalten sein.

Die Probenflüssigkeit ist üblicherweise eine im wesentlichen wässerige Flüssigkeit, insbesondere eine Körperflüssigkeit. Beispiele sind Urin, interstitielle Gewebsflüssigkeit und Blut.

Typische Analyten, die bestimmt werden können sind beispielsweise Glucose, Bilirubin, Ketone, pH-Wert, Proteine und Cholesterin.

Während bei den konventionellen colorimetrischen Testsystemen (Teststreifen, Biosensoren) pro Testelement nur eine Bestimmung möglich ist, erlauben die reagenzfreien Elemente der erfindungsgemäßen Testsysteme simultan oder sequentiell eine Mehrfachbestimmung, beispielsweise Glucose- und Cholesterin-Test innerhalb eines Testelementes.

In einer besonderen Ausführungsform der Erfindung ist die flüssige Probe Blut oder interstitielle Gewebsflüssigkeit und der Analyt Glucose.

Die spektroskopische Messung erfolgt üblicherweise in Transmission durch die Mikrokapillarwand hindurch.

Nach Einsaugen der Probenflüssigkeit, beispielsweise interstitielle Gewebsflüssigkeit, erfolgt die qualitative und/oder quantitative Bestimmung des Analyten, beispielsweise Glucose, auf spektroskopischem Wege vorzugsweise unmittelbar in der Mikrokapillare, wobei die Mikrokapillare quasi als Küvette fungiert.

Im Hinblick auf eine praktikable Handhabung sowie eine spektroskopische Auswertung sind die erfindungsgemäßen Mikrokapillartestsysteme in ein entsprechendes Format zu integrieren.

Für die folgenden Beispiele, in denen das Einsaugverhalten verschiedener kommerziell erhältlicher GC-Mikrokapillarsäulen untersucht wurde, wurden als Mikrokapillare jeweils etwa 1 cm lange Mikrokapillarsäulenstücke der Fa. Varian verwendet.

### Beispiele

### Beispiel 1: Einsaugverhalten von GC-Mikrokapillarsäulen mit verschiedenen Innenbeschichtungen (stationären Phasen)

Es wurde jeweils ein etwa 1 cm langes Mikrokapillarstück der folgenden Säulentypen der Fa. Varian verwendet:
CP-Sil 5 CB: stationäre Phase: 100% dimethylpolysiloxan
CP-Wax 52 CB: stationäre Phase: polyethylenglykol
CP-Wax 52 CB: stationäre Phase: cyanopropylpolysiloxan

Als Testflüssigkeit diente eine Glucometer ENCORE™ Glucose control solution, die üblicherweise als Blutersatz zum Kontrollieren der Glucose-Teststreifen eingesetzt wird.

Es wurden folgende Einsaugergebnisse ermittelt:
CP-Sil 5 CB: Die Testflüssigkeit wurde nicht eingesaugt.
CP-Wax 52 CB: Die Testflüssigkeit wurde innerhalb weniger Sekunden eingesaugt.
CP-Wax 52 CB: Die Testflüssigkeit wurde innerhalb weniger Sekunden eingesaugt.

### Beispiel 2: Tensid-Beschichtung einer hydrophoben GC-Mikrokapillarsäule

Eine GC-Mikrokapillarsäule mit hydrophober stationärer Phase (CP-Sil 5 CB, Fa. Varian), die wie in Beispiel 1 erwähnt die Probeflüssigkeit nicht einsaugt, wurde in folgender Weise mit einem Tensid beschichtet:

Tensidlösung: 1%ige Lösung von Phospholipon 90 G (Fa. Rhone-Poulenc) in isoPropanol.

Eine etwa 1 cm lange CP-Sil 5 CB-Mikrokapillarsäule wurde im Vakuum mit der Tensidlösung beschichtet und anschließend getrocknet (dynamische Beschichtung, s. K. Grob: Making and Manipulating Capillary Columns for Gas Chromatography).

Einsaugtest mit ENCORE™ Control Solution: Ein 2 cm langes Mikrokapillarstück war in weniger als 1 sec vollgesaugt.

## Patentansprüche

1. Verwendung von spektroskopischen Methoden zur qualitativen und/oder quantitativen Bestimmung eines oder mehrerer Analyten in einer mit einer Mikrokapillare aufgenommenen Probe.

2. Verwendung gemäß Anspruch 1, **dadurch gekennzeichnet, dass** die Mikrokapillare auf der inneren Oberfläche eine Beschichtung trägt.

3. Verwendung gemäß Anspruch 1 oder 2 zur Bestimmung von Glucose in Blut oder interstitieller Gewebsflüssigkeit.

4. Verwendung gemäß einem der vorstehenden Ansprüche, **dadurch gekennzeichnet, dass** die Mikrokapillare aus Glas oder Quarz besteht.

5. Verwendung gemäß einem der vorstehenden Ansprüche, **dadurch gekennzeichnet, dass** der Innendurchmesser der Mikrokapillare 500 um oder weniger beträgt.

6. Verwendung von Infrarot- oder Raman-Spektroskopie gemäß Anspruch 1.

7. Testsystem zur qualitativen und/oder quantitativen Bestimmung eines oder mehrerer Analyten in einer flüssigen Probe, bei dem die Probenaufnahme mit einer Mikrokapillare und die Bestimmung des oder der Analyten mit einer spektroskopischen Methode erfolgt.

8. Testsystem gemäß Anspruch 7, **dadurch gekennzeichnet, dass** die Bestimmung des oder der Analyten in der Mikrokapillare erfolgt.

9. Testsystem gemäß Anspruch 7 oder 8, **dadurch gekennzeichnet, dass** das über die Mikrokapillare aufzunehmende Probenvolumen kleiner als 3 µl ist.

10. Testsystem gemäß einem der Anspruch 7 bis 9, **dadurch gekennzeichnet, dass** die Bestimmung des oder der Analyten Infrarot- oder Raman-Spektroskopie erfolgt.
